# EUROPÄISCHE PATENTSCHRIFT

(11) **EP 2 829 295 B1**
(45) Veröffentlichungstag und Bekanntmachung des Hinweises auf die Patenterteilung: **12.07.2017**
(21) Anmeldenummer: 14166960.6
(22) Anmeldetag: 05.05.2014
(51) Int. Cl.: A61M 5/32

(54) **Medizinische Injektionsvorrichtung**
Medical injection device
Dispositif d'injection médicale

(30) Priorität: 24.07.2013 DE 102013214442
(43) Veröffentlichungstag der Anmeldung: 28.01.2015
(73) Patentinhaber: RAUMEDIC AG, 95213 Münchberg (DE)
(72) Erfinder: Jakob, Thomas, 95111 Rehau (DE); Maag, Sebastian, 95448 Bayreuth (DE); Festel, Tobias, 95234 Sparneck (DE); Braun, Thomas, 95032 Hof (DE); Skaper, Frank, 95191 Leupoldsgrün (DE)
(74) Vertreter: Rau, Schneck & Hübner Patentanwälte Rechtsanwälte PartGmbB

(56) Entgegenhaltungen:
- EP-A1- 1 790 367
- WO-A2-2004/103431
- US-A- 4 720 285

## Beschreibung

Die Erfindung betrifft eine medizinische Injektionsvorrichtung mit einer Injektionseinrichtung.

Medizinische Injektionsvorrichtungen sind bekannt aus der EP 460 914 B1, der EP 707 860 B1, der US 4,838,871, der EP 692 271 B1, der US 4,944,397, der US 4,982,842, der US 5,232,455, der US 5,139,489, der US 5,154,285, der US 5,277,311, der US 5,232,454, der US 5,312,367, der US 5,342,322, der US 5,423,765, der US 5,643,219, der WO 1991/009639 A2, der EP 862 920 B1, der EP 885 621 B1, der EP 1 525 016 B1, der EP 1 568 321 A1, der EP 1 587 419 B1, der EP 1 592 346 B1, der US 5,584,816 und der US 5,632,732. Aus der DE 600 32 171 T2 ist eine Injektionsspritze mit einer Nadelumhüllung bekannt.

Die WO 2004/103431 A2 beschreibt einen Nadelschutz für Spritzennadeln. Die EP 1 790 367 A1 beschreibt ein medizinisches Werkzeug mit einer Injektionsnadel mit einer Schutzkappe. Die US 4,720,285 beschreibt eine Injektionsspritze mit einer Schutzkappe.

Es ist eine Aufgabe der vorliegenden Erfindung, eine Injektionsvorrichtung der eingangs genannten Art derart weiterzubilden, dass eine intuitiv handhabbare und betriebssichere Injektionsvorrichtung resultiert.

Diese Aufgabe ist erfindungsgemäß gelöst durch eine medizinische Injektionsvorrichtung mit einer Verdrehschutzeinrichtung nach Anspruch 1.

Zum Lösen der Schutzkappe von der Injektionskanüle kann ein Benutzer einerseits an der Verdrehschutzeinrichtung und andererseits an der Schutzkappe angreifen. Beim Abdrehen der Schutzkappe zum Lösen der Schutzkappe vom Mündungs- und Verbindungsabschnitt ist dann ein unerwünschtes Verdrehen des Behälters relativ zum Mündungs- und Verbindungsabschnitt im Bereich der Steckverbindung verhindert. Dies stellt sicher, dass die Steckverbindung beim Abdrehen der Schutzkappe nicht ungewollt gelöst wird, so dass eine Sterilität eines Inneren des Behälters gesichert ist. Bei der Steckverbindung kann es sich um eine Konusverbindung handeln. Bei der Steckverbindung kann es sich um eine Luerlock-Verbindung handeln. Die Steckverbindung kann rotationssymmetrisch so ausgeführt sein, dass ein Verdrehen des Mündungs- und Verbindungsabschnitts relativ zum kanülenseitigen Ende des Behälters um eine Kanülenachse möglich ist. Die Steckverbindung kann mit mindestens einem Rastabschnitt versehen sein, so dass die Verbindung über einen Formschluss gesichert ist. Die Verdrehschutzeinrichtung der medizinischen Injektionsvorrichtung ist über einen Formschluss-Adapter mit der Injektionseinrichtung verbunden. Ein solcher Formschluss-Adapter stellt eine verdrehgeschützte Verbindung der Verdrehschutzeinrichtung mit der Injektionseinrichtung sicher. Der Formschluss-Adapter ist mit der sonstigen Verdrehschutzeinrichtung ebenfalls verdrehgesichert verbunden. Der Formschluss-Adapter kann ein Bauteil der Verdrehschutzeinrichtung sein.

Eine axiale Überdeckung der Steckverbindung durch die Verdrehschutzeinrichtung nach Anspruch 2 stellt sicher, dass die Steckverbindung von außen her unzugänglich ist, was ebenfalls dazu beiträgt, ein unerwünschtes Lösen der Steckverbindung zu vermeiden.

Dies gilt besonders durch einen Formschluss-Adapter nach Anspruch 3.

Eine Gestaltung der Verdrehschutzeinrichtung nach Anspruch 4 sorgt für einen besonders sicheren Verdrehschutz des Formschluss-Adapters relativ zum Mündungs- und Verbindungsabschnitt. Das Niederhaltemittel kann durch eine Niederhaltehülse, beispielsweise durch eine Teleskophülse, gebildet sein, die bei einer Montage der Verdrehschutzeinrichtung über den Formschluss-Adapter geschoben wird. Durch das Niederhaltemittel wird die mindestens eine Formschluss-Zunge drehfest mit dem Mündungs- und Verbindungsabschnitt verbunden. Die sonstige Verdrehschutzeinrichtung kann fest mit der mindestens einen Formschluss-Zunge verbunden sein. Es kann eine Mehrzahl von um eine axiale Längsachse des Mündungs- und Verbindungsabschnitts und damit der gesamten Injektionseinrichtung umlaufenden Formschluss-Zungen vorgesehen sein. Eine Anzahl der Formschluss-Zungen kann auf eine Breite und eine Anzahl von Umfangsrippen des Mündungs- und Verbindungsabschnitts abgestimmt sein.

Eine Tragring-Gestaltung des Formschluss-Adapters nach Anspruch 5 führt zu einem besonders stabilen Aufbau und zu einem effektiven Verdrehschutz. Alternativ kann der Formschluss-Adapter auch C-förmig ausgestaltet sein, so dass er von der Seite her auf den Mündungs- und Verbindungsabschnitt aufgeschoben und insbesondere auf diesen aufgerastet werden kann. Dies erleichtert eine Montage des Formschluss-Adapters.

Eine Ausführung nach Anspruch 6 zum Niederhalten der mindestens einen Formschluss-Zunge führt zu einem besonders sicheren Verdrehschutz des Formschluss-Adapters relativ zum Mündungs- und Verbindungsabschnitt. Das Niederhalte-Gegenstück kann durch eine äußere Axialrippe am Formschluss-Adapter ausgeführt sein.

Eine Mehrkomponenten-Spritzgussgestaltung nach Anspruch 7 erweitert die Möglichkeiten zur Gestaltung der Bauteile der Stechschutzeinrichtung. Das Mehrkomponenten-Spritzgussteil kann als Zweikomponenten-Spritzgussteil oder auch als Spritzgussteil mit mehr als zwei Komponenten, beispielsweise drei Komponenten, vier Komponenten, fünf Komponenten oder noch mehr Komponenten, ausgeführt sein. Es können weichere Kunststoffe mit härteren Kunststoffen kombiniert werden. Weichere Kunststoffe können beispielsweise für einen Griffabschnitt der Stechschutzeinrichtung oder für durch Anlage an Gegenkomponenten wirkende Anformbauteile zum Spielausgleich und/oder zur Schaffung bzw. Erhöhung einer Reibschlusswirkung zwischen dem jeweiligen Anformbauteil und der jeweiligen Gegenkomponente genutzt werden.

Eine Weichkomponente des Mehrkomponenten-Spritzgussteils kann beispielsweise aus einem oder mehrerer thermoplastischer Elastomere (TPE), aus Polyurethan oder aus Silikon gefertigt sein. Eine Hartkomponente des Mehrkomponenten-Spritzgussteils kann aus Polypropylen, aus Polyethylen, aus ABS (Acrylnitril-Butadien-Styrol), aus einem Thermoplasten auf Basis von Methylmethacrylat, Acrylnitril, Butadien und Styrol (MABS), aus Polyoxymethylen (POM), aus Polybutylenterephthalat (PBT) oder auch als Blendsystem, also als Mischung, auf Basis von Polyolefinen sowie Polyamid gefertigt sein.

Die Ausführung nach dem Anspruch 8 gibt eine vorteilhafte Variante beim Einsatz von Mehrkomponenten-Spritzguss-Bauteilen an.

Eine Mehrkomponenten-Spritzguss-Gestaltung des Formschluss-Elements und des Formschluss-Element-Tragkörpers ermöglicht es, das mindestens eine Formschluss-Element, das als Axialrippe ausgeführt sein kann, aus einem im Vergleich zum Formschluss-Element-Tragkörper weicheren Kunststoffmaterial auszuführen, so dass ein Reibschluss der Formschluss-Elemente am inneren Mündungs- und Verbindungsabschnitt verbessert ist. Dies verbessert die Verdrehsicherheit des Formschluss-Adapters relativ zum Mündungs- und Verbindungsabschnitt.

Ausführungsbeispiele der Erfindung werden nachfolgend anhand der Zeichnung näher erläutert. In dieser zeigen:
- Fig. 1: eine medizinische Injektionsvorrichtung mit einer Injektionseinrichtung und einer TeleskopStechschutzeinrichtung in montiertem und auslieferungsfertigem Zustand;
- Fig. 2: die Injektionsvorrichtung nach Fig. 1 in benutzungsfertigem Zustand, bei dem eine ursprüngliche Schutzkappe von einer Injektionskanüle abgenommen ist;
- Fig. 3: die Injektionsvorrichtung nach Fig. 2 während einer Verlagerung der Teleskop-Stechschutzeinrichtung zwischen einer Injektionsstellung nach den Fig. 1 und 2, in der die Injektionskanüle zum Injizieren eines Mediums freilegbar (Fig. 1) oder freigelegt (Fig. 2) ist, und einer Schutzstellung, in der eine Kanülenspitze der Injetionskanüle in einer Schutzkomponente der Teleskop-Stechschutzeinrichtung versenkt angeordnet ist;
- Fig. 4: die Injektionsvorrichtung mit der TeleskopStechschutzeinrichtung in der Schutzstellung;
- Fig. 5: einen Teil-Längsschnitt durch die Injektionsvorrichtung mit aufgesetzter ursprünglicher Schutzkappe, wobei die Teleskop-Stechschutzeinrichtung in einer Stellung zwischen der Injektionsstellung und der Schutzstellung dargestellt ist;
- Fig. 6: eine Ausschnittsvergrößerung des Details VI in Fig. 5;
- Fig. 7: eine vergrößerte Ansicht eines Ausschnitts der Injektionsvorrichtung in der Schutzstellung;
- Fig. 8 bis 11: Momentanpositionen bei der Montage der Injektionsvorrichtung;
- Fig. 12: eine Explosionsdarstellung einer weiteren Ausführung einer medizinischen Injektionsvorrichtung mit einer Teleskop-Stechschutzeinrichtung;
- Fig. 13: die Injektionsvorrichtung nach Fig. 12 mit der Stechschutzeinrichtung in der Injektionsstellung;
- Fig. 14: die Injektionsvorrichtung nach Fig. 12 mit der Stechschutzeinrichtung in der Schutzstellung;
- Fig. 15: einen Formschluss-Adapter zur Formschlussverbindung der Stechschutzeinrichtung nach den Fig. 12 bis 14 mit der Injektionseinrichtung, ausgeführt als radial aufrastbarer, C-förmiger Adapter;
- Fig. 16: eine Teleskophülse der Stechschutzeinrichtung nach den Fig. 12 bis 14, die zwischen den FormschlussAdapter nach Fig. 15 und einer Schutz-Teleskophülse der Stechschutzeinrichtung angeordnet ist;
- Fig. 17: die Schutz-Teleskophülse der Stechschutzeinrichtung nach den Fig. 12 bis 14;
- Fig. 18: eine Explosionsdarstellung einer weiteren Ausführung einer Teleskop-Stechschutzeinrichtung für eine medizinische Injektionsvorrichtung;
- Fig. 19: eine Injektionsvorrichtung mit der Stechschutzeinrichtung nach Fig. 18 in der Injektionsstellung;
- Fig. 20: die Injektionsvorrichtung nach Fig. 18 mit der Stechschutzeinrichtung in der Schutzstellung;
- Fig. 21: einen Formschluss-Adapter zur Formschlussverbindung der Stechschutzeinrichtung nach den Fig. 18 bis 20 mit der Injektionseinrichtung;
- Fig. 22: eine Verbindungs-Teleskophülse der Stechschutzeinrichtung nach den Fig. 18 bis 20, die zwischen dem Formschluss-Adapter nach Fig. 21 und einer mittleren Teleskophülse der Stechschutzeinrichtung angeordnet ist;
- Fig. 23: eine mittlere Teleskophülse der Stechschutzeinrichtung nach den Fig. 18 bis 20, die zwischen der Verbindungs-Teleskophülse nach Fig. 22 und einer SchutzTeleskophülse der Stechschutzeinrichtung angeordnet ist;
- Fig. 24: die Schutz-Teleskophülse der Stechschutzeinrichtung nach den Fig. 18 bis 20;
- Fig. 25: einen ringförmigen Deckel für die Schutz-Teleskophülse nach Fig. 24;
- Fig. 26: einen axialen Längsschnitt durch die Stechschutzeinrichtung nach den Fig. 18 bis 20, dargestellt in der Schutzstellung;
- Fig. 27 bis 35: zu den Fig. 18 bis 26 ähnliche Darstellungen von Bauteilen einer weiteren Ausführung einer Stechschutzeinrichtung für eine Injektionseinrichtung;
- Fig. 36 bis 42: zu den Fig. 18 bis 24 ähnliche Darstellungen von Bauteilen einer weiteren Ausführung einer Stechschutzeinrichtung für eine Injektionseinrichtung; und
- Fig. 43: einen axialen Längsschnitt durch die Stechschutzeinrichtung nach den Fig. 36 bis 38, dargestellt in der Schutzstellung.

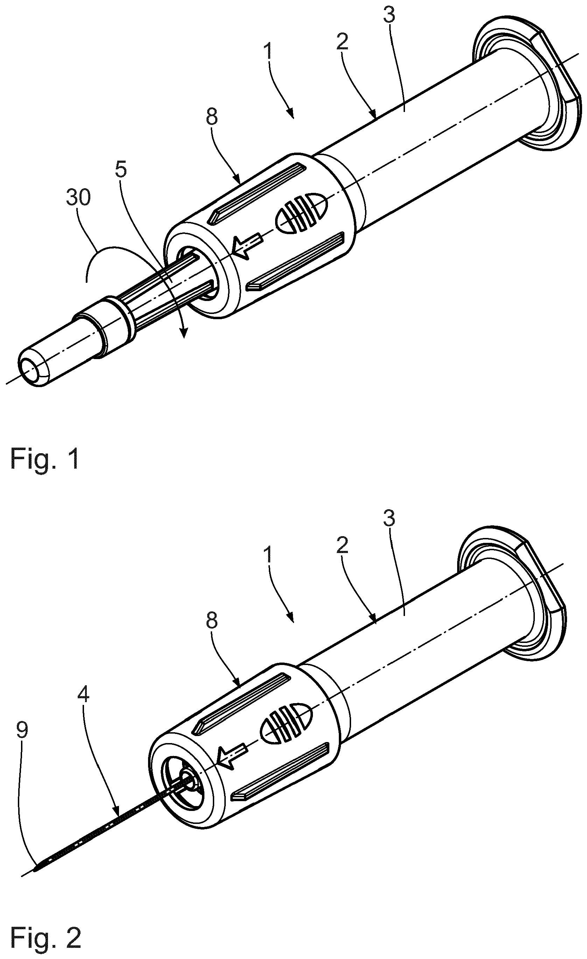

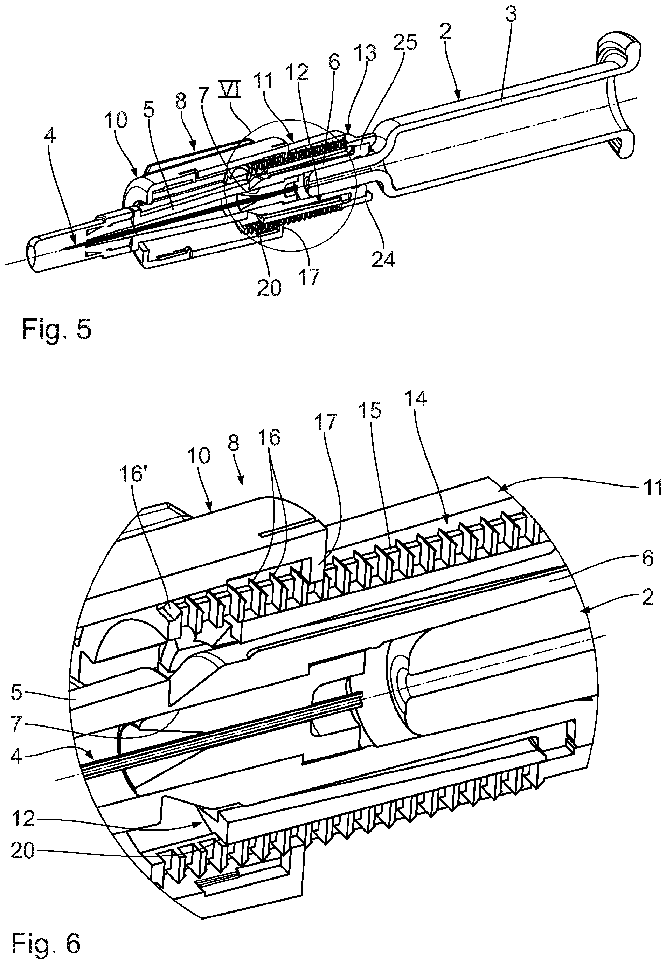

Fig. 1 bis 11 zeigen eine Ausführung einer medizinischen Injektionsvorrichtung 1. Die Injektionsvorrichtung 1 hat eine Injektionseinrichtung 2. Zu dieser gehört ein Behälter 3 für das zu injizierende Medium. Der Behälter 3 kann als Spritzenbehälter 3 zur Aufnahme eines Spritzenkolbens ausgeführt sein, der in der Zeichnung nicht dargestellt ist. Zur Injektionseinrichtung 2 gehört weiterhin eine Injektionskanüle 4, die in der Fig. 2 sichtbar ist und die in der Fig. 1 von einer ursprünglichen Schutzkappe 5 abgedeckt ist. Die ursprüngliche Schutzkappe 5 ist im ursprünglich ausgelieferten Zustand der Injektionsvorrichtung 1 nach Fig. 1 auf die Injektionskanüle 4 aufgesetzt und mit einem kanülenseitigen Ende des Behälters 3 axial verrastet. Zum Injizieren des Mediums kommuniziert die Injektionskanüle 4 mit dem Behälter 3 über einen kanülenseitigen Mündungs- und Verbindungsabschnitt 6 des Behälters 3, der in der Schnittdarstellung nach Fig. 5 sichtbar ist. Der Mündungs- und Verbindungsabschnitt 6 ist auf ein konisch zulaufendes Mündungsende eines Glaskörpers des Behälters 3 aufgeschoben und kann mit diesem zusätzlich formschlüssig und insbesondere rastend verbunden sein. Die Injektionskanüle 4, bei der es sich um eine metallische Kanüle handelt, ist mit dem Mündungsabschnitt 6 über eine Steck- bzw. Konusverbindung 7 verbunden. Bei einer nicht dargestellten Variante der Injektionsvorrichtung 1 ist die Konusverbindung 7 als Luerlock-Verbindung gestaltet. Eine Innenwand des Mündungsabschnitts 6 kann im Bereich der Steck- bzw. Konusverbindung 7 mit einer Außenwand eines Kanülenansatzes verrastet oder in sonstiger Weise formschlüssig verbunden sein.

Abgesehen von der Injektionskanüle 4 sind sämtliche Komponenten der Injektionsvorrichtung 1 aus Kunststoff gefertigt. Grundsätzlich kann auch die Injektionskanüle 4 aus Kunststoff gefertigt sein.

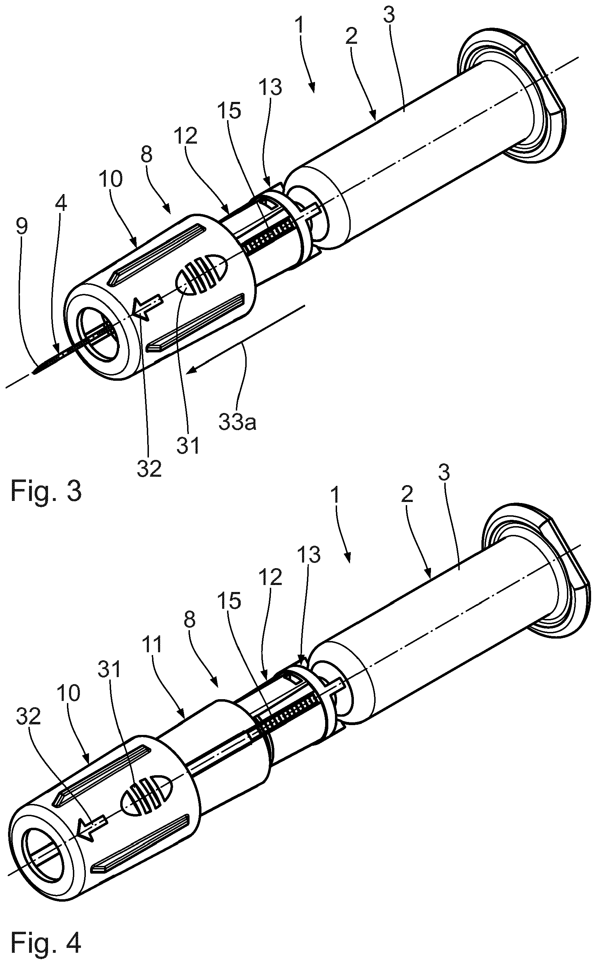

Die Injektionsvorrichtung 1 hat weiterhin eine Stechschutzeinrichtung 8. Diese ist verlagerbar zwischen einer in der Fig. 2 dargestellten Injektionsstellung, in der die Injektionskanüle 4 zum Beispiel zum subkutanen oder intravenösen Injizieren des Mediums freilegbar ist, und einer in der Fig. 4 dargestellten Schutzstellung, in der eine Kanülenspitze 9 der Injektionskanüle 4 in einer Schutzkomponente 10 der Stechschutzeinrichtung 8 versenkt angeordnet ist.

Die Stechschutzeinrichtung 8 umgibt den Mündungsabschnitt 6 in Form einer Hülse und hat mindestens zwei Teleskophülsen. Bei der Ausführung nach den Fig. 1 bis 11 hat die Stechschutzeinrichtung 8 insgesamt drei Teleskophülsen 10, 11 und 12, wobei eine dieser drei Teleskophülsen, die Schutz-Teleskophülse 10, die Schutzkomponente der Stechschutzeinrichtung 8 darstellt. Die Schutz-Teleskophülse 10 ist gleichzeitig die äußerste der drei Teleskophülsen 10 bis 12 der Stechschutzeinrichtung 8. Eine innerste der drei Teleskophülsen, die Verbindungs-Teleskophülse 12, ist über einen Formschluss-Adapter 13 mit der Injektionseinrichtung 2 verbunden. Zwischen der innersten Teleskophülse 12 und der äußersten Teleskophülse 10 der Stechschutzeinrichtung 8 liegt die Teleskophülse 11 als weitere Teleskophülse der Stechschutzeinrichtung 8.

In der Injektionsstellung sind die Teleskophülsen 10 bis 12 vollständig übereinander geschoben. In der Injektionsstellung überdeckt die Stechschutzeinrichtung 8 die Konusverbindung 7 axial, so dass diese von außen her nicht zugänglich ist. In der Schutzstellung sind die Teleskophülsen 10 bis 12 relativ zueinander teleskopiert.

Die Fig. 5 und 6 zeigen Details der Injektionsvorrichtung 1 und insbesondere Details der Stechschutzeinrichtung 8.

Zum sichernden Festlegen der Schutzkomponente, also der äußersten Schutz-Teleskophülse 10, in der Schutzstellung dient eine Sicherungseinrichtung in Form einer Schutz-Rastanordnung 14. Diese hat Rast-Zahnreihen 15 mit hintereinander längs der mittleren Teleskophülse 11 und der inneren Verbindungs-Teleskophülse 12 angeordneten Rastzähnen 16. Jede der Teleskophülsen 11, 12 hat zwei in Umfangsrichtung um die Längsachse der Injektionsvorrichtung einander gegenüberliegend angeordnete äußere Rast-Zahnreihen 15. Die beiden Rast-Zahnreihen 15 der mittleren Teleskophülse 11 sind relativ zu den beiden Zahnreihen 15 der inneren Verbindungs-Teleskophülse 12 um 90° in Umfangsrichtung um die Längsachse der Injektionsvorrichtung 1 versetzt.

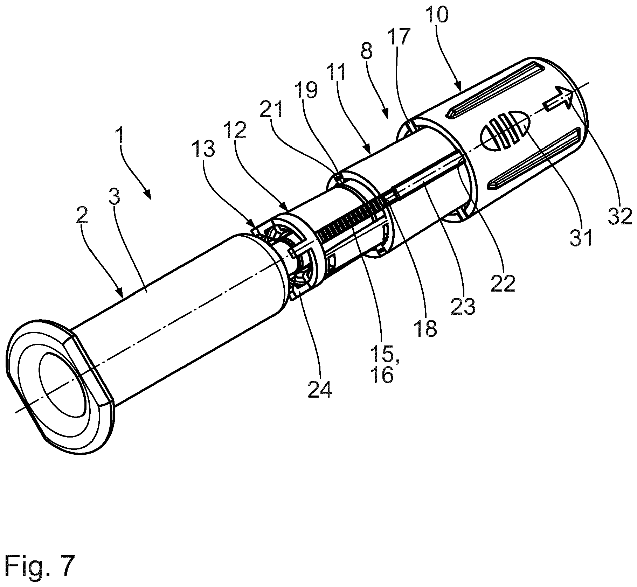

In die Rastzähne 16 greift jeweils ein Gegenrastkörper 17 der äußeren Schutz-Teleskophülse 10 bzw. ein Gegenrastkörper 18 (vgl. Fig. 7) der mittleren Teleskophülse 11 ein. Die Rastzähne 16 haben im axialen Längsschnitt der Injektionsvorrichtung ein Sägezahnprofil mit einer Vorzugsrichtung, um eine Einweg-Umstellung der Teleskophülsen 10, 11 von der Injektionsstellung in die teleskopierte Schutzstellung zu gewährleisten. Ein Rastzahn 16' (vgl. Fig. 6), der einer maximal teleskopierten Relativstellung der zugeordneten Teleskophülsen 10, 11 entspricht, hat im Axialschnitt eine genau gegenläufige Vorzugsrichtung, um die End-Teleskopierstellung, also die Schutzstellung der Stechschutzeinrichtung 8 zu definieren.

Die Rastzähne 16 sind einstückig an die jeweilige Teleskophülse 11, 12 angeformt.

Zur Gewährleistung einer Teleskop-Führung und gleichzeitig eines Verdrehschutzes zwischen zwei benachbarten der drei Teleskophülsen 10 bis 12, also zwischen den Teleskophülsen 10, 11 einerseits und den Teleskophülsen 11, 12 andererseits dienen Nut/Feder-Führungseinrichtungen 19 der Stechschutzeinrichtung 8.

Fig. 7 zeigt eine Nut 20 einer der Nut/Feder-Führungseinrichtungen 19, die in einer äußeren Mantelwand der inneren Verbindungs-Teleskophülse 12 als axiale Längsnut ausgeführt ist. In diese Nut 20 greift eine komplementär ausgeführte Feder 21 ein, die in einer Innenwand der mittleren Teleskophülse 11 nach innen vorstehend ausgeführt ist. Die Feder 21 der mittleren Teleskophülse 11, die mit der Nut 20 der inneren Teleskophülse 12 führend zusammenwirkt, ist durch die inneren Enden der Rastzähne 16 der mittleren Teleskophülse 11 gebildet.

Eine weitere Nut/Feder-Führungseinrichtung 19 ist gebildet durch axiale Längsnuten 22 in der inneren Mantelwand der äußeren Schutz-Teleskophülse 10 und hierzu komplementäre Federn 23, die radial nach außen überstehend in der äußeren Mantelwand der mittleren Teleskophülse 11 ausgeführt sind. Jeweils zwei gleichartige Nut/Feder-Führungseinrichtungen 19 sind einander gegenüberliegend in Bezug auf die Längsachse der Injektionsvorrichtung 1 angeordnet. Jeweils bezogen auf eine der Teleskophülsen 10 bis 12 wechselt sich in Umfangrichtung um die Längsachse der Injektionsvorrichtung 1 jeweils in 90°-Schritten eine Rastkomponente der Schutz-Rastanordnung 14 mit einer Komponente der Nut/Feder-Führungseinrichtung 19 ab.

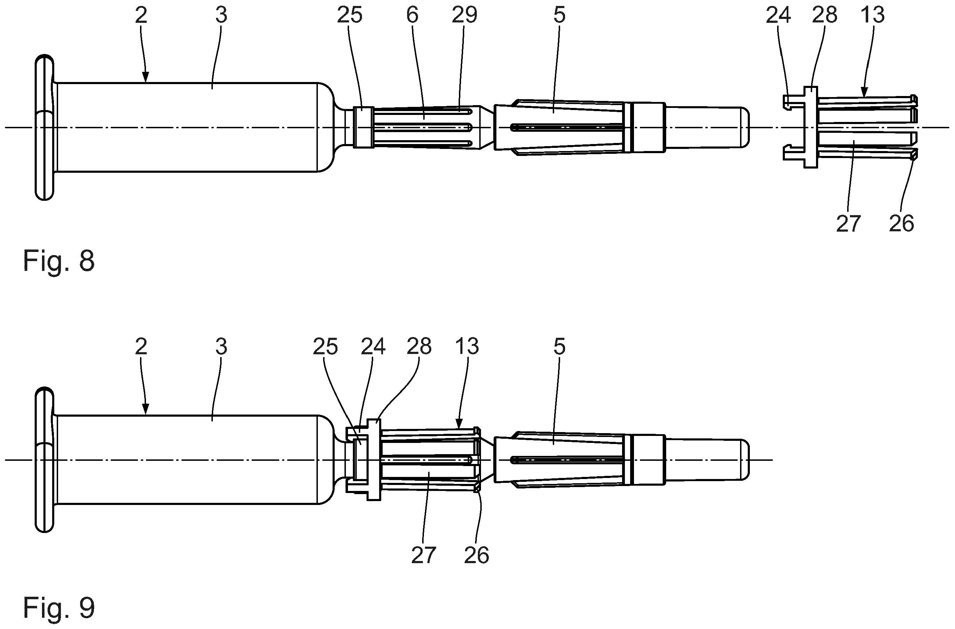

Die Fig. 8 bis 11 zeigen Momentanpositionen bei der Montage der Injektionsvorrichtung 1. Der Formschluss-Adapter 13 ist mithilfe von Rasthaken 24 rastend mit dem Behälter 3 der Injektionseinrichtung 2 verbunden. Hierzu hintergreifen die Rasthaken 24 einen Rastbund 25 des Behälters 3, der am Übergang zum Mündungsabschnitt 6 angeordnet ist.

Die innere Verbindungs-Teleskophülse 12 ist axial mit dem Formschluss-Adapter 13 über eine Mehrzahl von Rastkörpern 26 verbunden, die an freien Enden von Rastzungen 27 des Formschluss-Adapters 13 angeformt sind. Die Rastzungen 27 erstrecken sich in axialer Richtung und sind an einen gemeinsamen Tragring 28 des Formschluss-Adapters 13 angeformt. Insgesamt erhält hierdurch der Formschluss-Adapter 13 die Form einer axial aufsteckbaren Adapterhülse. Ein Abstand zwischen zwei in Umfangrichtung um die Längsachse der Injektionsvorrichtung 1 benachbarten Rastzungen 27 und die Anzahl der Rastzungen 27 ist abgestimmt auf eine Breite und eine Anzahl von axial verlaufenden Umfangsrippen 29, die außen an den Mündungsabschnitt 6 des Behälters 3 angeformt sind. Jeweils eine der Rastzungen 27 fügt sich bei aufgestecktem Formschluss-Adapter 13 zwischen zwei Benachbarten der Umfangsrippen 29, sodass ein Verdrehschutz des Formschluss-Adapters 13 relativ zum Behälter 3, nämlich zum Mündungs- und Verbindungsabschnitt 6 des Behälters 3,gegeben ist. Eine Innenwand der inneren Verbindungs-Teleskophülse 12 ist mit Axialstrukturen versehen, die in der Zeichnung nicht näher dargestellt sind und die bei auf den Formschluss-Adapter 13 aufgerasteter innerer Verbindungs-Teleskophülse 12 für einen Verdrehschutz der inneren Verbindungs-Teleskophülse 12 relativ zum Formschluss-Adapter 13 sorgen. Die inneren Axialstrukturen der Verbindungs-Teleskophülse 12 greifen dabei zwischen jeweils benachbarten Rastzungen 27 des Formschluss-Adapters 13 ein.

Bei aufgerasteter innerer Verbindungs-Teleskophülse 12 hintergreifen die Rastkörper 26 einen komplementär hierzu gebildeten Rastbund der Verbindungs-Teleskophülse 12, was in der Zeichnung nicht näher dargestellt ist.

Zum formschlüssigen Festlegen der Schutz-Teleskophülse 10, also der Schutzkomponente der Stechschutzeinrichtung 8, an der Injektionseinrichtung 2 in der Injektionsstellung dient eine Injektions-Verbindungsanordnung, die als Injektions-Rastanordnung ausgeführt ist. Rastkomponenten dieser Injektions-Rastanordnung sind einerseits die äußeren Kanten der freien Enden der Rasthaken 24 des Formschluss-Adapters 13 und andererseits die in der Injektionsstellung diese hintergreifenden Gegenrastkörper 17 der Schutz-Teleskophülse 10. Diese Injektions-Rastanordnung 17, 24 ist durch Ausklinken der Gegenrastkörper 17 aus dem Hintergriff zu den Rastzungen 24 überwindbar. Diese Überwindung kann durch einen definierten Druck auf die Stechschutzeinrichtung 8 erreicht werden.

Die Injektionsvorrichtung 1 wird folgendermaßen montiert: Zunächst liegt die Injektionseinrichtung 2 in einer handelsüblichen Auslieferungs-Gestaltung vor, die in der Fig. 8 wiedergegeben ist. Die Stechschutzeinrichtung 8 mit den Hülsen 10 bis 12 ist in der Injektionsstellung vormontiert, in der die Teleskophülsen vollständig übereinandergeschoben sind. Auf die Injektionseinrichtung 2 wird dann der Formschluss-Adapter 13 von der Kanülenseite der Injektionseinrichtung 2 her mit führenden Rasthaken 24 aufgeschoben, bis die Rasthaken 24 den Rastbund 25 des Behälters 3 rastend hintergreifen (vgl. Fig. 9). Anschließend wird die vorgefertigte Stechschutzeinrichtung 8 mit den drei ineinander gesteckten und rastend miteinander verbundenen Teleskophülsen 10 bis 12, ebenfalls von der Kanülenseite der Injektionseinrichtung 2 her auf diese aufgeschoben, bis einerseits die innere Verbindungs-Teleskophülse 12 auf den Formschluss-Adapter 13 aufgerastet ist, wobei die Rastzungen 27 radial zwischen die Umfangsrippen 29 zum Verdrehschutz gedrückt werden, und andererseits die Injektions-Rastanordnung 17, 24 zum Verrasten kommt. Die innere Verbindungs-Teleskophülse 12 wird in Umfangsrichtung so orientiert auf den Formschluss-Adapter 13 aufgeschoben, dass die inneren Axialstrukturen der Verbindungs-Teleskophülse 12 zwischen die Rastzungen 27 des Formschluss-Adapters 13 eingreifen. Bei vollständig aufgeschobener innerer Verbindungs-Teleskophülse 12 kommt ein führender Anschlagsbund der Verbindungs-Teleskophülse 12 in Anschlag an eine zugewandte Stirnwand des Tragrings 28 des Formschluss-Adapters 13.

Innere Strukturen der Verbindungs-Teleskophülse 12 dienen gleichzeitig als Niederhaltemittel zum Halten der Rastzungen 27 zwischen den Umfangsrippen 29 des Mündungsabschnitts 6 der Injektionseinrichtung 2.

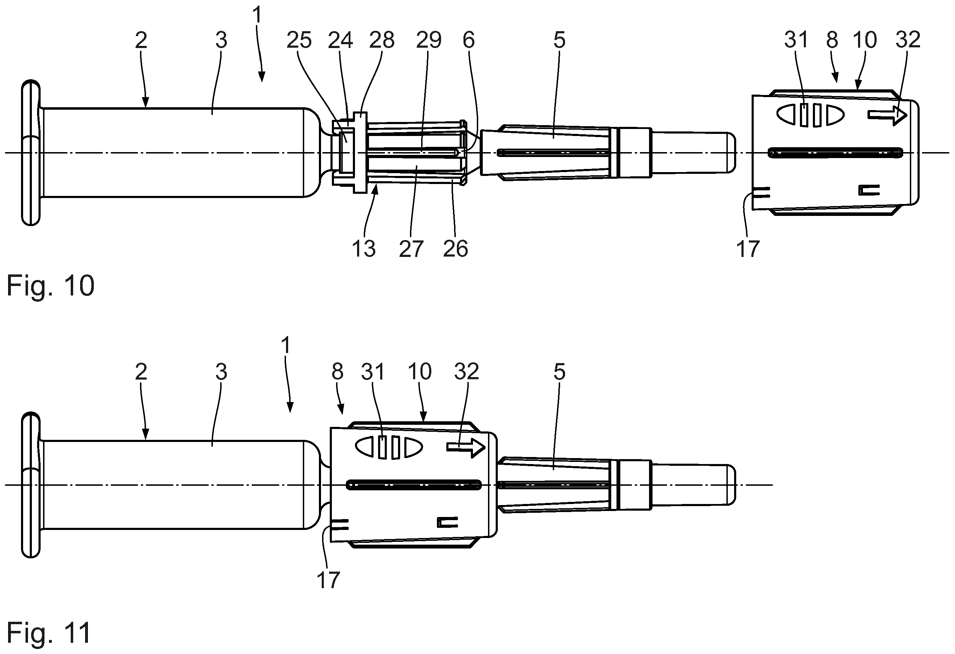

Nach der Montage liegt die Injektionsvorrichtung 1 mit der Stechschutzeinrichtung 8 in der Injektionsstellung und der ursprünglich schon montierten Schutzkappe 5 über der Injektionsnadel 4 vor, wie in den Fig. 1 und 11 dargestellt. Durch die verschiedenen Verdrehschutzkomponenten sind die insgesamt vier Bauteile 10 bis 13 der Stechschutzeinrichtung 8 zueinander verdrehgesichert und auch die gesamte Stechschutzeinrichtung 8 ist zur Injektionseinrichtung 2 verdrehgesichert.

Die Injektionsvorrichtung 1 wird folgendermaßen benutzt: Zunächst wird die Schutzkappe 5 von der Injektionskanüle 4 entfernt, was durch eine Abdrehbewegung geschieht (vgl. Pfeil 30 in der Fig. 1). Beim Abdrehen ist durch die über den Außenumfang des Behälters 3 überstehende Querschnittsgestaltung der Schutz-Teleskophülse 10 gesichert, dass der Benutzer zum Abdrehen der Schutzkappe 5 die Injektionsvorrichtung 1 außen an der Schutz-Teleskophülse 10 ergreift. Hierzu hat die Schutz-Teleskophülse 10 axial verlaufende Längsrippen, die verhindern, dass sich beim Abdrehen der Schutzkappe 5 die Schutz-Teleskophülse 10 unerwünscht zwischen den Fingern des Benutzers dreht. Da alle Bauteile der Stechschutzeinrichtung 8 zueinander und auch der Formschluss-Adapter 13 zum Mündungsabschnitt 6 verdrehgesichert sind, ist bei einer Relativverdrehung der Schutzkappe 5 gegen die Stechschutzeinrichtung 8 in Richtung des Pfeils 30 (oder in Gegenrichtung hierzu) gewährleistet, dass sich die Schutzkappe 5 tatsächlich gewünscht vom Mündungsabschnitt 6 abdreht. Nach diesem Abdrehen kann die Schutzkappe 5 von der Injektionskanüle 4 abgezogen werden.

Die Injektionsvorrichtung 1 liegt dann im gebrauchsfertigen Zustand vor, der in der Fig. 2 dargestellt ist. Zum Verlagern der Stechschutzeinrichtung 8 in die Schutzstellung (vgl. hierzu die Fig. 3 bis 7) wird zunächst in einem Druckbereich 31, der auf der äußeren Schutz-Teleskophülse 10 markiert ist, von beiden Seiten her Druck auf die Schutz-Teleskophülse 10 ausgeübt. Hierdurch klinken die Gegenrastkörper 17 von den Rasthaken 24 aus und die äußere Schutz-Teleskophülse 10 kann in Richtung des dort aufgebrachten Pfeils 32 axial relativ zu der Teleskophülse 11 teleskopiert werden (vgl. auch Pfeil 33a in der Fig. 3). Dabei rattert der Gegenrastkörper 17 über die Rastzähne 16 der Schutz-Rastanordnung 14, bis die Endstellung des Gegenrastkörpers 17 vor dem endseitigen Rastzahn 16' der mittleren Teleskophülse 11 erreicht ist. Anschließend teleskopiert auch die mittlere Teleskophülse 11 relativ zur inneren Verbindungs-Teleskophülse 12, wobei die Gegenrastkörper 18 der mittleren Teleskophülse 11 über die Rastzähne 16 der inneren Verbindungs-Teleskophülse 12 rattern, bis auch hier die Endstellung des Gegenrastkörpers 18 in Anlage wiederum zum endseitigen Rastzahn der inneren Verbindungs-Teleskophülse 12 erreicht ist. Die Stechschutzeinrichtung 8 liegt dann in der komplett teleskopierten Schutzstellung nach Fig. 4 vor. In dieser ist die Kanülenspitze 9 der Injektionskanüle 4 in der Schutz-Teleskophülse 10 vollständig versenkt angeordnet, sodass ein sicherer Stechschutz gewährleistet ist. Eine zerstörungsfreie Überführung der Stechschutzeinrichtung 8, ausgehend von der Schutzstellung nach Fig. 4, sodass die Kanülenspitze 9 wieder frei wird, ist für den Benutzer aufgrund der Einweg-Charakteristik der zugeordneten Rastanordnungen 14 nicht möglich.

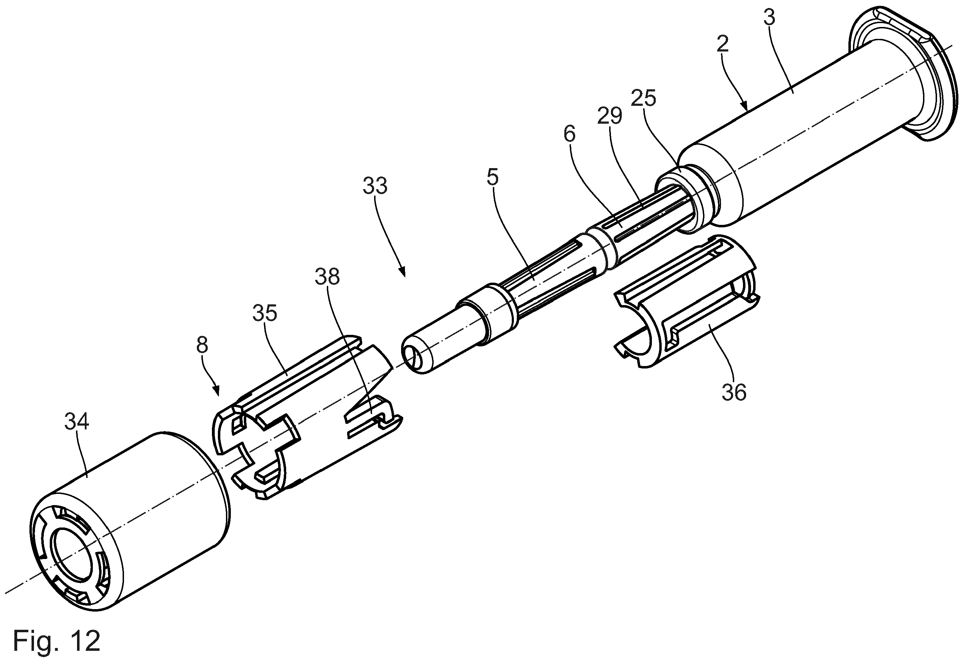

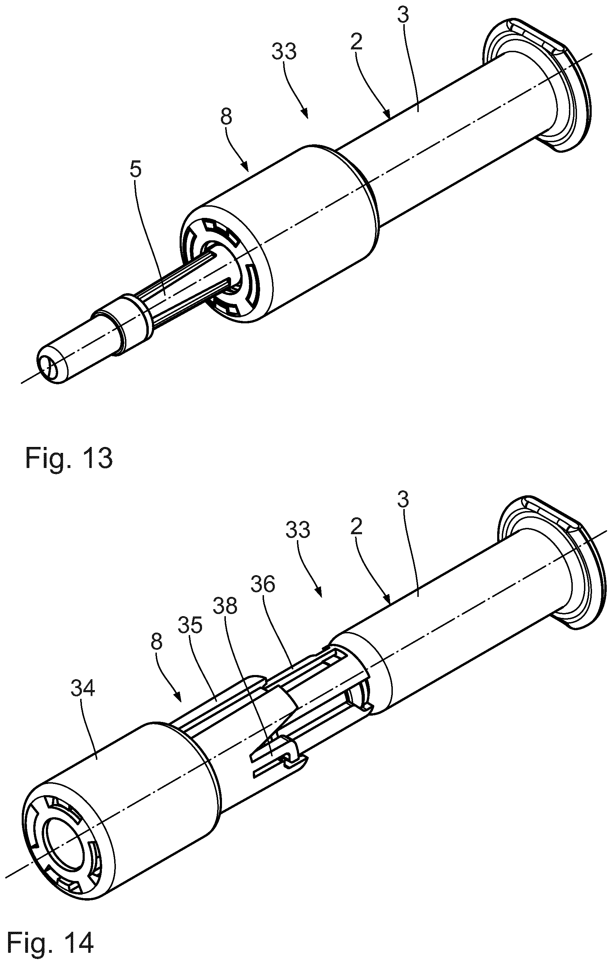

Anhand der Fig. 12 bis 17 wird nachfolgend eine weitere Ausführung einer Injektionsvorrichtung 33 erläutert. Komponenten und Funktionen, die denjenigen entsprechen, die vorstehend unter Bezugnahme auf die Fig. 1 bis 11 bereits beschrieben wurden, tragen die gleichen Bezugsziffern und werden nicht nochmals im Einzelnen diskutiert.

Die Stechschutzeinrichtung 8 der Injektionsvorrichtung 33 nach den Fig. 12 bis 17 hat wiederum drei Teleskophülsen, nämlich eine äußere Schutz-Teleskophülse 34, deren Funktion der Schutz-Teleskophülse 10 der Ausführung nach den Fig. 1 bis 11 entspricht, eine mittlere Teleskophülse 35, deren Funktion der Teleskophülse 11 der Ausführung nach den Fig. 1 bis 11 entspricht, und eine innere Verbindungs-Teleskopkomponente 36, die gleichzeitig einen Formschluss-Adapter zur Formschlussverbindung der Stechschutzeinrichtung 8 mit der Injektionseinrichtung 2 darstellt. Die Verbindungs-Teleskopkomponente 36 vereint also die Funktionen der inneren Teleskophülse 12 und des Formschluss-Adapters 13 der Ausführung nach den Fig. 1 bis 11.

Die Verbindungs-Teleskopkomponente 36 ist als radial aufrastbarer, C-förmiger Adapter ausgeführt. Die Verbindungs-Teleskopkomponente 36 wird radial auf den Mündungsabschnitt 6 des Behälters 3 aufgerastet, wobei zur axialen Sicherung der Verbindungs-Teleskopkomponente 36 der Rastbund 25 des Mündungsabschnitts 6 in einem Umfangsbereich von der Verbindungs-Teleskopkomponente 36 hintergriffen wird.

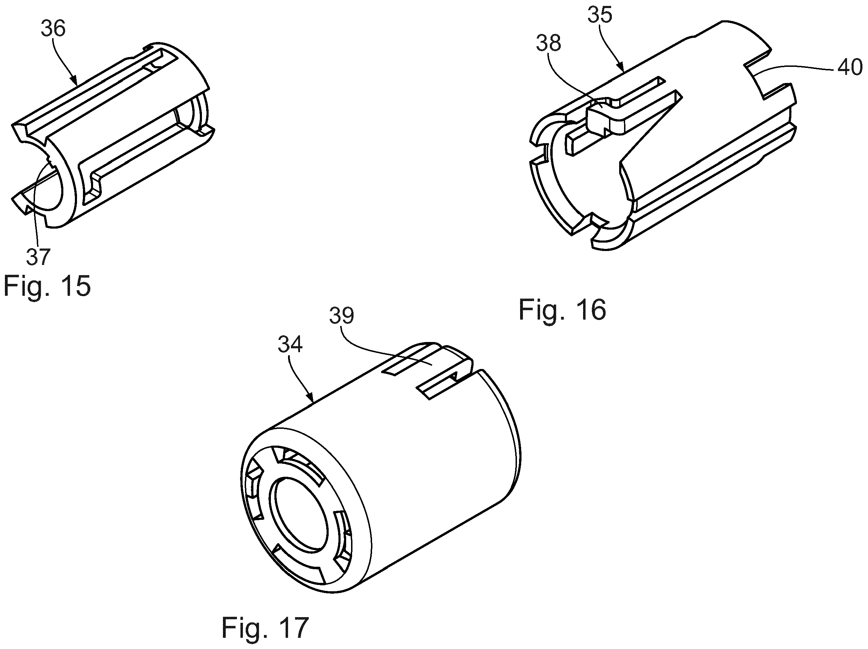

Um einen höheren Reibschluss zwischen der Verbindungs-Teleskopkomponente 36 und dem Mündungsabschnitt 6 des Behälters 3 der Injektionseinrichtung 2 und damit insbesondere einen Verdrehschutz zu gewährleisten, sind innere Rippen 37 der Verbindungs-Teleskopkomponente 36, die in montiertem Zustand mit dem Mündungsabschnitt 6 zwischen dessen Umfangsrippen 29 zur Anlage kommen, aus weicherem Kunststoffmaterial ausgeführt als der sonstige Grundkörper der Verbindungs-Teleskopkomponente 36. Die Rippen 37 können an den Grundkörper der Verbindungs-Teleskopkomponente 36 beispielsweise durch Mehrkomponenten-Technik, insbesondere durch 2K-Technik, angeformt sein. Von den inneren Rippen 37 ist in der Fig. 15 genau eine Rippe 37 angedeutet. Tatsächlich liegen mehrere, z. B. fünf, Rippen 37 in Umfangsrichtung abgestimmt auf die Umfangsabstand der Umfangsrippen 29 gleichbeabstandet vor.

Die mittlere Teleskophülse 35 (vgl. auch Fig. 16) ist mit der Verbindungs-Teleskopkomponente 36 über eine radial wirkende Rastverbindung verbunden. Hierzu hat die mittlere Teleskophülse 35 eine Federzunge 38, die in eine entsprechende Rastausnehmung der Verbindungs-Teleskopkomponente 36 eingreift.

Die äußere Schutz-Teleskophülse 34 hat ebenfalls eine Federzunge 39, die zur rastenden Verbindung eine entsprechende Rastaufnahme in der mittleren Teleskophülse 35 bzw. der Verbindungs-Teleskopanordnung 36 hintergreift. Die Federzunge 39 und die in der Injektionsstellung zugeordnete Rastaufnahme bilden somit die Injektions-Verbindungsanordnung zum formschlüssigen Festlegen der Teleskop-Schutzhülse 34 an der Injektionseinrichtung 2 in der Injektionsstellung.

In der Schutzstellung der Stechschutzeinrichtung 8 in der Ausführung nach den Fig. 12 bis 17 hintergreift die Rastzunge 39 eine Rastaufnahme 40, die in der mittleren Teleskophülse 35 ausgeführt ist. Hierdurch und durch eine entsprechende Rastverbindung der mittleren Teleskophülse 35 an der Verbindungs-Teleskopkomponente 36 ist eine Schutz-Rastanordnung zum rastenden Festlegen der Schutz-Teleskophülse 34 in der Schutzstellung gegeben.

Abgesehen von den vorstehend erläuterten Unterschieden entspricht die Montage und auch die Benutzung der Injektionsvorrichtung 33 dem, was vorstehend unter Bezugnahme auf die Injektionsvorrichtung 1 bereits erläutert wurde.

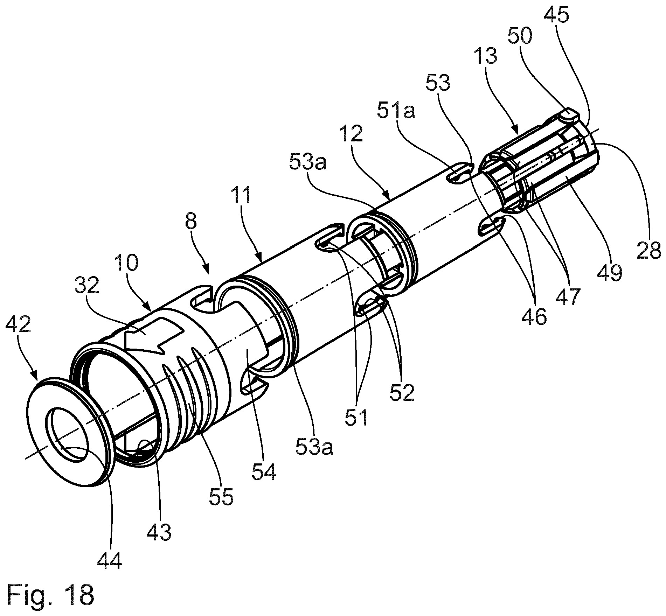

Anhand der Fig. 18 bis 26 wird nachfolgend eine weitere Ausführung einer Injektionsvorrichtung 41 erläutert. Komponenten und Funktionen, die denjenigen entsprechen, die vorstehend unter Bezugnahme auf die Injektionsvorrichtungen 1 und 33 bereits erläutert wurden, tragen die gleichen Bezugsziffern und werden nicht nochmals im Einzelnen diskutiert.

Die Injektionsvorrichtung 41 hat ebenfalls eine Teleskop-Stechschutzeinrichtung 8, die mit einer Verbindungs-Teleskophülse 12, einem Formschluss-Adapters 13, einer mittleren Teleskophülse 11 und einer Schutz-Teleskophülse 10 grundsätzlich so aufgebaut ist wie die Schutzeinrichtung 8 der Injektionsvorrichtung 1. Unterschiede ergeben sich in Details der Rastverbindungen und der Führungsgestaltungen. Die Rastverbindungen sind bei der Injektionsvorrichtung 41 als Axial-Rastverbindungen ausgeführt.

Die Schutz-Teleskophülse 10 ist bei der Stechschutzeinrichtung 8 der Injektionsvorrichtung 41 zweiteilig ausgeführt und hat neben der eigentlichen Teleskophülse noch einen ringförmigen Deckel 42. Über einen Außenumfang ist der Deckel 42 in eine innere Umfangsnut 43 in einem äußeren Endbereich der Schutz-Teleskophülse 10 eingerastet. Durch einen äußeren Umfang des Deckels 42 und die innere Umfangsnut 43 ist eine Ringschnappverbindung zwischen dem Deckel 42 und der Schutz-Teleskophülse 10 gebildet. Der Deckel 42 dient dazu, eine von außen zugängliche Öffnungsweite der Schutz-Teleskophülse 10 auf eine Durchgangsöffnung 44 mit im Vergleich zum Innendurchmesser der sonstigen Schutz-Teleskophülse 10 verringertem Durchmesser zu reduzieren.

Bei der Montage der Stechschutzeinrichtung 8 nach den Fig. 18 bis 26 wird zunächst die mittlere Teleskophülse 11 von der Seite der inneren Umfangsnut 43 her in die noch ohne Deckel vorliegende Schutz-Teleskophülse 10 eingesetzt. Anschließend wird von der gleichen Seite her die Verbindungs-Teleskophülse 12 in die mittlere Teleskophülse 11 eingesetzt. Sodann wird der Deckel 42 in die innere Umfangsnut 43 eingerastet. Von der gegenüberliegenden Seite wird dann der Formschluss-Adapter 13 in die Verbindungs-Teleskophülse 12 eingesetzt.

Die so vormontierte Stechschutzeinrichtung kann dann auf die Injektionseinrichtung 2 aufgeschoben werden. Dies geschieht, bis ein Anlagebund 45 des Formschluss-Adapters 13 am Rastbund 25 der Injektionseinrichtung 2 (vgl. z. B. Fig. 8) anliegt.

Beim weiteren Aufschieben der Stechschutzeinrichtung 8 nach den Fig. 18 bis 26 auf die Injektionseinrichtung 2 in Richtung auf den Behälter 3 zu wird die Verbindungs-Teleskophülse 12 axial zum dann axial am Rastbund 25 festgelegten Formschluss-Adapter 13 verschoben, bis Rasthaken 46, die an die Verbindungs-Teleskophülse 12 angeformt sind, den Rastbund 25 des Mündungsabschnitts 6 der Injektionseinrichtung 2 hintergreifen. Bei der Injektionsvorrichtung 41 dienen also nicht Rasthaken am Formschluss-Adapter 13, sondern die Rasthaken 46 an der Verbindungs-Teleskophülse 12 zum formschlüssigen Festlegen unter anderem der Schutz-Teleskophülse 10 als Schutzkomponente der Stechschutzeinrichtung 8 an der Injektionseinrichtung 2 in der Injektionsstellung.

Auch bei der Injektionsvorrichtung 41 ist ein Verdrehschutz zwischen der Stechschutzeinrichtung 8, die also auch eine Verdrehschutzeinrichtung ist, und dem Mündungsabschnitt 6 der Injektionseinrichtung 2 gegeben. Hierzu hat der Formschluss-Adapter 13 der Injektionsvorrichtung 41 wiederum Verdrehschutz-Zungen 47, die den Rastzungen 27 der Ausführung nach den Fig. 1 bis 11 entsprechen. Die Verdrehschutz-Zungen 47 verlaufen axial und sind über den Tragring 28 des Formschluss-Adapters 13 der Injektionsvorrichtung 41 miteinander verbunden. Montiert sind die Verdrehschutz-Zungen 47 jeweils zwischen zwei benachbarten, axial verlaufenden Umfangsrippen 29 des Mündungsabschnitts 6 der Injektionseinrichtung 2 aufgenommen.

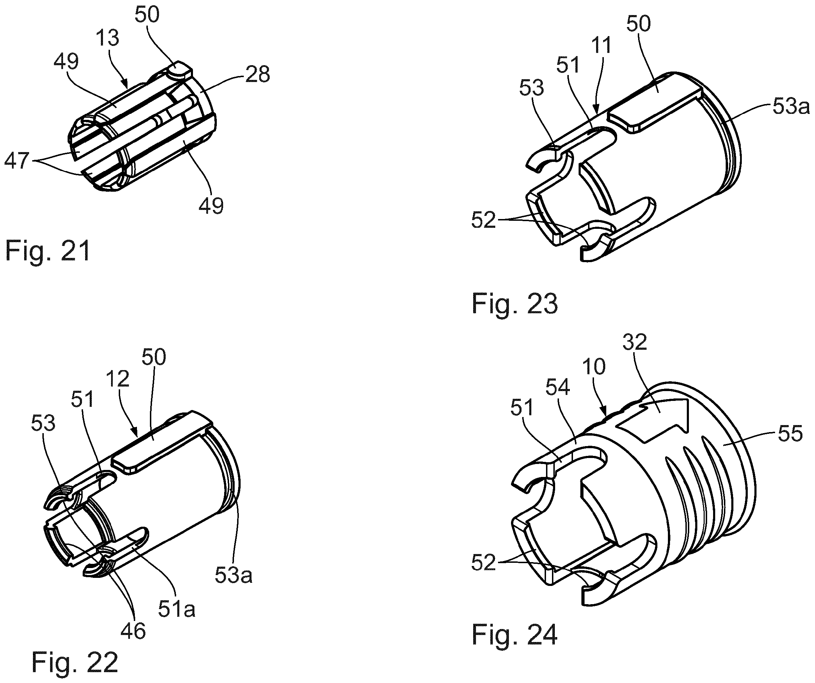

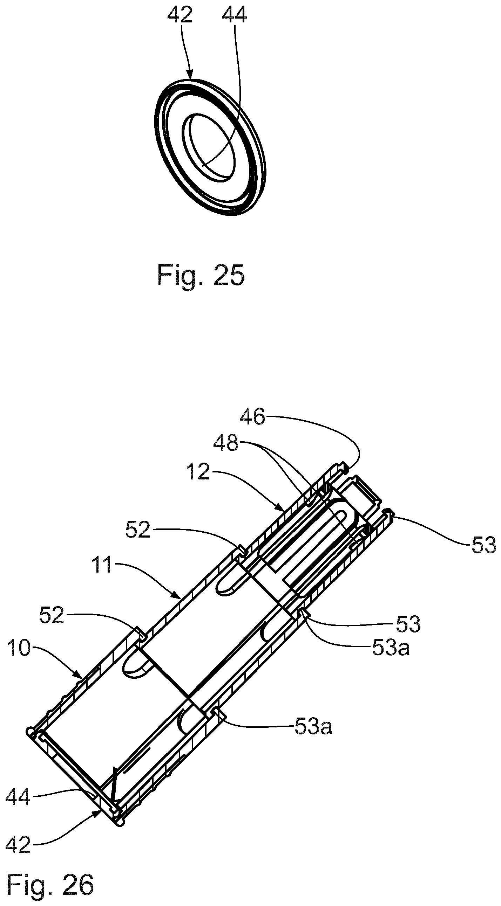

Zum Halten der Verdrehschutz-Zungen 47 zwischen den Umfangsrippen 29 dient ein an der Verbindungs-Teleskophülse 12 ausgebildetes Niederhaltemittel. Dieses ist gebildet durch insgesamt vier innere Axialrippen, von denen zwei im Axialschnitt nach Fig. 26 sichtbar sind. Die Axialrippen 48 sind jeweils um 90° in Umfangsrichtung versetzt an einer Innenwand der Verbindungs-Teleskophülse 12 angeformt. Jede der Axialrippen 48 wirkt mit einem Niederhalte-Gegenstück am Formschluss-Adapter 13 zum Niederhalten jeweils einer Verdrehschutz-Zunge 47 zusammen. Die Niederhalte-Gegenstücke sind durch äußere Axialrippen 49 am Formschluss-Adapter 13 ausgebildet (vgl. Fig. 21).

Der Formschluss-Adapter 13 ist an der Verbindungs-Teleskophülse 12 und jeweils zwei aneinander anliegende Teleskophülsen 12, 11, 10 sind gegeneinander jeweils durch Verdrehsicherungen gegen eine Relativverdrehung um die Längsachse der Stechschutzeinrichtung 8 gesichert. Diese Verdrehsicherung ist wiederum gebildet durch äußere Federn 50 an jeweils einer der Komponenten 13, 12, 11, die mit hierzu komplementären inneren Axialnuten 51 in den jeweils benachbarten, außenanliegenden Teleskophülsen 12, 11, 10 verdrehsichernd zusammenwirken.

Die Federn 50 dienen gleichzeitig als Anschläge, die mit axial verlaufenden Ausnehmungen 51 a als Anschläge zur Vorgabe einer axialen Endposition der Verbindungs-Teleskophülse 12 relativ zum Formschluss-Adapter 13 bei der rastenden Verbindung der Stechschutzeinrichtung 8 mit dem Rastbund 25 des Mündungsabschnitts 6 über die Rasthaken 46 der Verbindungs-Teleskophülse 12 zusammenwirken.

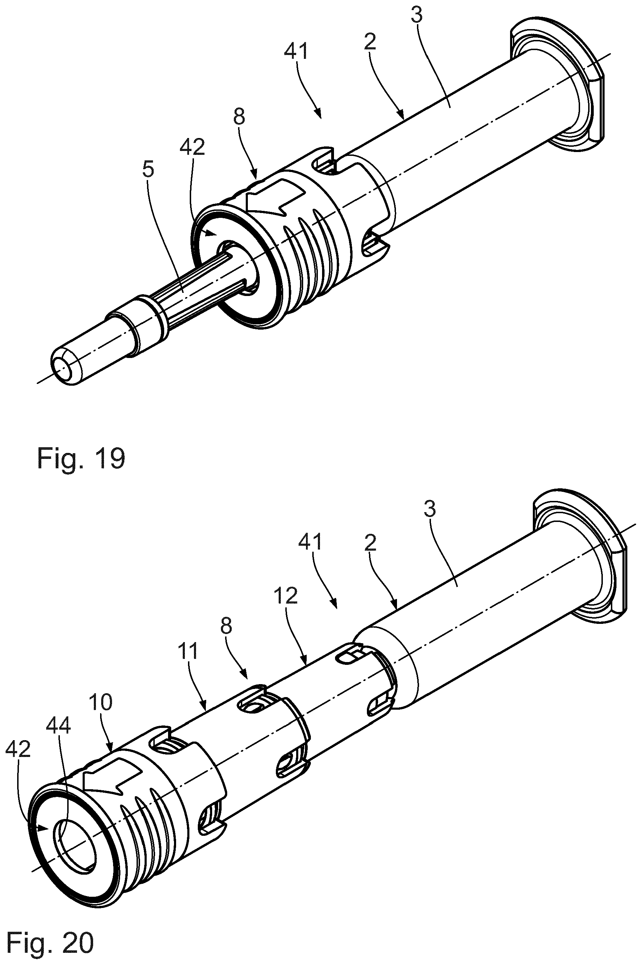

Vergleichbar zu den Rasthaken 46 der Verbindungs-Teleskophülse 12 haben auch die mittlere Teleskophülse 11 und die Schutz-Teleskophülse 10 vergleichbare, radial wirkende Rasthaken 52. Genau wie die Rasthaken 46 sind auch die Rasthaken 52 in Umfangsrichtung jeweils um 90° versetzt zueinander angeordnet. In der Injektionsstellung, beispielsweise nach Fig. 19, liegen die Rasthaken 46, 52 benachbarter Teleskophülsen 12, 11, 10 genau übereinander. Die Rasthaken 52 der mittleren Teleskophülse 11 hintergreifen dabei hierzu komplementäre Ausnehmungen 53 in der Außenseite der Rasthaken 46. Die Rasthaken 52 der Schutz-Teleskophülse 10 hintergreifen entsprechende Ausnehmungen 53 in der Außenseite der Rasthaken 52 der mittleren Teleskophülse 11.

Die Rasthaken 52 der mittleren Teleskophülse 11 einerseits und der Schutz-Teleskophülse 10 andererseits wirken in der Schutzstellung der Stechschutzeinrichtung 8 (vgl. z. B. Fig. 20 und 26) mit äußerem Umfangsnuten 53a einerseits in der Verbindungs-Teleskophülse 12 und andererseits in der mittleren Teleskophülse 11 zusammen.

Bei der Überführung der Teleskophülsen 11, 10 von der zusammengeschobenen Injektionsstellung in die ausgefahrene Schutzstellung gleiten die Rasthaken 52 zwischen den jeweiligen Gegen-Ausnehmungen 53 und den Umfangsnuten 53a. Damit bei der Überführung der Teleskophülsen 11, 10 in die Schutzstellung eine gleichmäßige Kraftentfaltung auf die Rasthaken 52 erfolgt, erweitern sich die Teleskophülsen 12, 11 zwischen den jeweiligen Gegenausnehmungen 53 und den jeweiligen Umfangsnuten 53a konisch.

Die Schutz-Teleskophülse 10 ist als 2K-Spritzgussteil ausgeführt. Neben einem Tragkörper 54 hat die Schutz-Teleskophülse 10 einen Griffabschnitt 55. Der Tragkörper 54 einerseits und der Griffabschnitt 55 andererseits sind als unterschiedliche Spritzguss-Komponenten des 2K-Bauteils ausgeführt. Als 2K-Kunststoffe können ABS (Acrylnitril-Butadien-Styrol) für eine Hartkomponente, also beispielsweise für den Tragkörper 54, und TPE (thermoplastisches Elastomer) für eine Weichkomponente, beispielsweise für den Quickabschnitt 55, zum Einsatz kommen. Auch eine andere Anzahl von Komponenten können bei einem solchen Mehrkomponenten-Spritzgussteil zum Einsatz kommen, beispielsweise drei oder mehr Komponenten aus verschiedenen und insbesondere aus verschieden harten Kunststoffen.

Durch die Ausbildung der Schutz-Teleskophülse 10 als 2K-Spritzgussteil ist im Bereich des Griffabschnitts 55 eine griffigere Haptik der Schutz-Teleskophülse 10 gewährleistet.

Auch die als Niederhaltemittel dienenden Axialrippen 48 der Verbindungs-Teleskophülse 12 können aus einem zur sonstigen Verbindungs-Teleskophülse 12 anderem Kunststoffmaterial gefertigt sein und die Axialrippen 48 können mittels 2K-Spritzgustechnik an einen sonstigen Tragkörper der Verbindungs-Teleskophülse 12 angeformt sein.

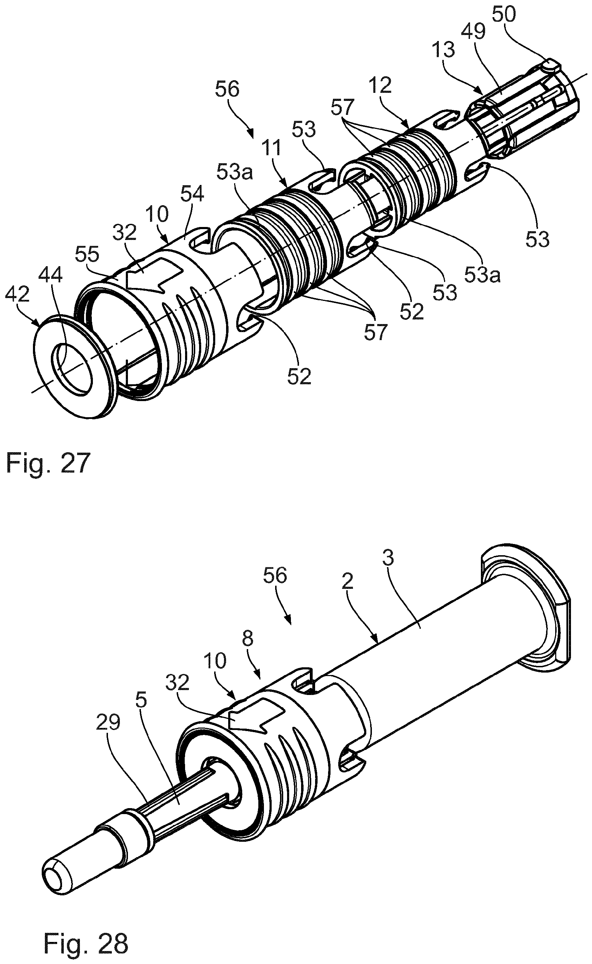

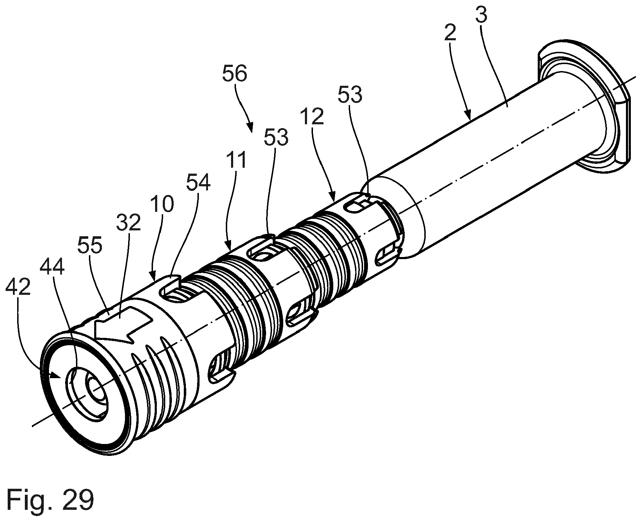

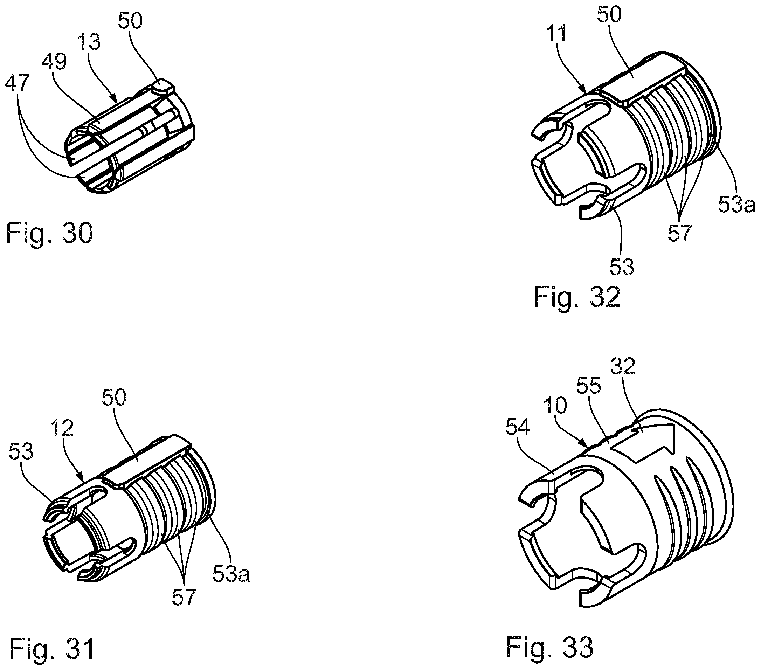

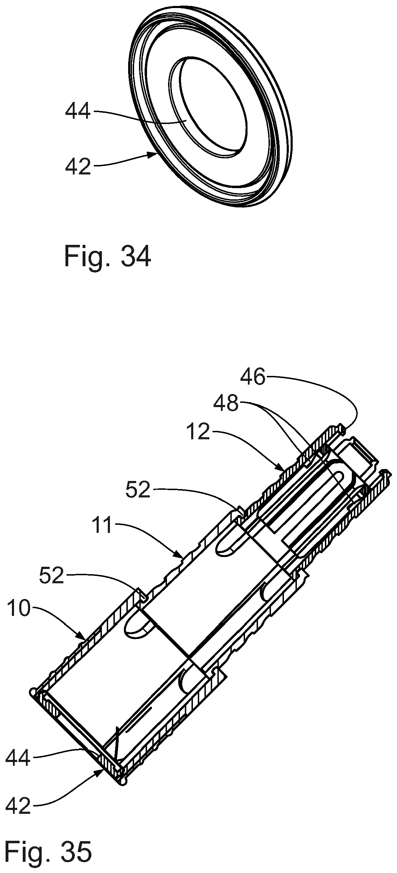

Anhand der Fig. 27 bis 35 wird nachfolgend eine weitere Ausführung einer Injektionsvorrichtung 56 erläutert. Komponenten und Funktionen, die denjenigen entsprechen, die vorstehend unter Bezugnahme auf die Injektionsvorrichtungen 1, 33 und 41 und insbesondere unter Bezugnahme auf die Injektionsvorrichtung 41 bereits erläutert wurden, tragen die gleichen Bezugsziffern und werden nicht nochmals im Einzelnen diskutiert.

Zwischen den Gegenausnehmungen 53 und den Umfangsnuten 53a haben die Verbindungs-Teleskophülse 12 und die mittlere Teleskophülse 11 jeweils drei Rast-Zwischenstufen 57. Beim Verlagern der Schutz-Teleskophülse 10 und der mittleren Teleskophülse 11 zwischen der Injektionsstellung und der Schutzstellung rasten die jeweiligen Rasthaken 52 der Schutz-Teleskophülse 10 und der mittleren Teleskophülse 11 auf ihrem Verlagerungsweg zwischen den jeweiligen Gegenausnehmungen 53 und den jeweiligen Umfangsnuten 53a über die Rast-Zwischenstufen 57. Es ergibt sich auf dem Weg der beiden Teleskophülsen 10, 11 zwischen der Injektionsstellung und der Schutzstellung eine haptische Rückmeldung für den Benutzer hinsichtlich des bereits zurückgelegten Verstellweges.

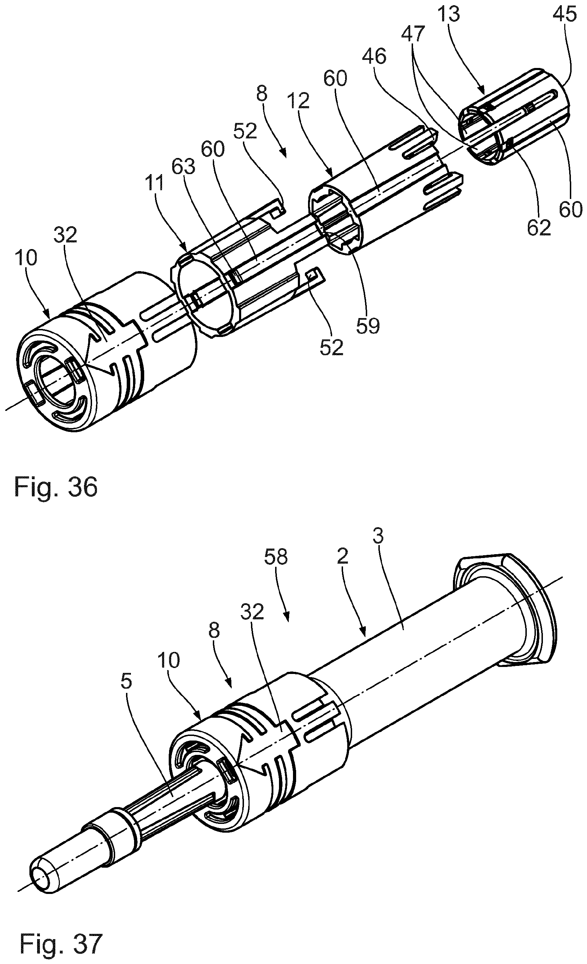

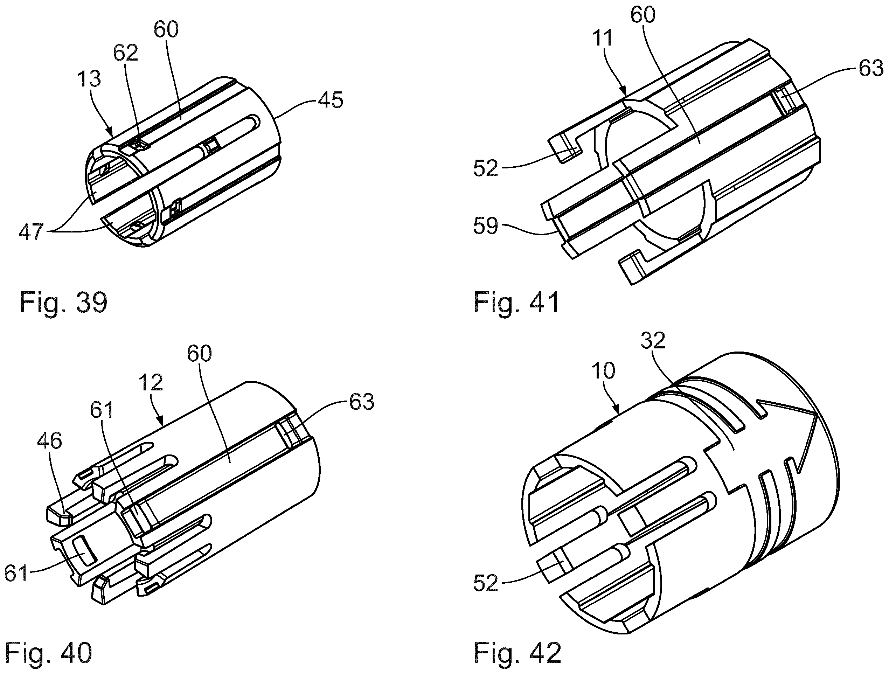

Anhand der Fig. 36 bis 43 wird nachfolgend eine weitere Ausführung einer Injektionsvorrichtung 58 erläutert. Komponenten und Funktionen, die denjenigen entsprechen, die vorstehend unter Bezugnahme auf die Injektionsvorrichtungen 1, 33, 41 und 56 bereits erläutert wurden, tragen die gleichen Bezugsziffern und werden nicht nochmals im Einzelnen diskutiert.

Auch die Injektionsvorrichtung 58 ist, ähnlich wie die Injektionsvorrichtung 1, vierteilig ausgeführt mit einem inneren Formschluss-Adapter 13, einer Verbindungs-Teleskophülse 12, einer mittleren Teleskophülse 11 und einer äußeren Schutz-Teleskophülse 10.

Die Funktion von Rasthaken 46 und 52 der Teleskophülsen 12, 11 und 10 bei der Injektionsvorrichtung 58 ähnelt der Injektionsvorrichtung 41. Die Teleskophülsen 11 und 10 haben jeweils zwei einander gegenüberliegende, in Umfangsrichtung also um 180° zueinander versetzt angeordnete Rasthaken 52. Bei montierter Injektionsvorrichtung 8 sind die Rasthaken 52 der mittleren Teleskophülse 11 relativ zu den Rasthaken 52 der Schutz-Teleskophülse 10 um 90° in Umfangsrichtung versetzt, ähnlich wie beim Konzept der Gegenrastkörper und der Rastzähne bei der Injektionsvorrichtung 1.

Bei der Montage wird zunächst die mittlere Teleskophülse 11 in die äußere Schutz-Teleskophülse 10 in Richtung des Pfeils 32 eingeschoben, bis die Rasthaken 52 der äußeren Schutz-Teleskophülse 10 Gegenausnehmungen 59 der mittleren Teleskophülse 11 hintergreifen, die am Ende von axialen Führungsbahnen 60 in einer Außenwand der mittleren Teleskophülse 11 ausgeführt sind.

Anschließend wird die Verbindungs-Teleskophülse 12 ebenfalls in Richtung des Pfeils 32 in die mittlere Teleskophülse 11 eingeschoben. Dies geschieht solange, bis die Rasthaken 52 der mittleren Teleskophülse 11 in Ausnehmungen 61 der Verbindungs-Teleskophülse 12 zu liegen kommen, die wiederum an Ende von axialen Führungsbahnen 60 in einer Außenwand der Verbindungs-Teleskophülse 12 ausgeführt sind.

Sodann wird der Formschluss-Adapter 13, wiederum in Richtung des Pfeils 32, in die Verbindungs-Teleskophülse 12 eingeschoben, bis die Rasthaken 46 der Verbindungs-Teleskophülse 12 von außen in Ausnehmungen 62 des Formschluss-Adapters 13 eingreifen. Die Ausnehmungen 62 sind wiederum in axialen Führungsbahnen 60 des Formschluss-Adapters 13 ausgeführt. In dieser vormontierten Position sind die Hülsen 11 und 12 praktisch vollständig in der äußeren Schutz-Teleskophülse 10 angeordnet. Der Formschluss-Adapter 13 steht mit dem größten Teil einer Axialerstreckung zwischen den Ausnehmungen 62 und dem Anlagebund 45 über die ineinandergeschobenen Teleskophülsen 10 bis 12 über.

Bei der Montage der vormontierten Stechschutzeinrichtung 8 mit der Injektionseinrichtung 2 wird die Stechschutzeinrichtung 8 mit führendem Formschluss-Adapter 13 auf den Mündungsabschnitt 6 der Injektionseinrichtung 2 aufgeschoben, bis der Anlagebund 45 am Rastbund 25 des Mündungsabschnitts 6 anschlägt. Anschließend werden die drei ineinandergeschobenen Teleskophülsen 10 bis 12 in axialer Richtung weiter auf den Behälter 3 zu verlagert, wobei die Rasthaken 46 der Verbindungs-Teleskophülse 12 aus den Ausnehmungen 62 des Formschluss-Adapters 13 ausrücken und zunächst längs der Führungsbahnen 60 gleiten und sodann den Rastbund 25 zur Sicherung der Stechschutzeinrichtung 8 an der Injektionseinrichtung 2 hintergreifen. Gleichzeitig sorgen wiederum Niederhaltemittel dafür, dass die Verdrehschutz-Zungen 47 des Formschluss-Adapters 13 zwischen benachbarten Umfangsrippen 29 des Mündungsabschnitts 6 zum Verdrehschutz der Stechschutzeinrichtung 8 an der Injektionseinrichtung 2 niedergehalten werden.

Das Zusammenwirken der Führungsbahnen 60 mit den zugeordneten Rasthaken 46, 52 dient als Verdrehschutz der Komponenten der Stechschutzeinrichtung 8 relativ zueinander. Eine weitere Verdrehsicherung ist durch Axialführungen jeweils um 90° versetzt zu den Rasthaken/Führungsbahnen-Gestaltungen gegeben.

Die Stechschutzeinrichtung 8 ist dann vorbereitet in der Injektionsstellung.

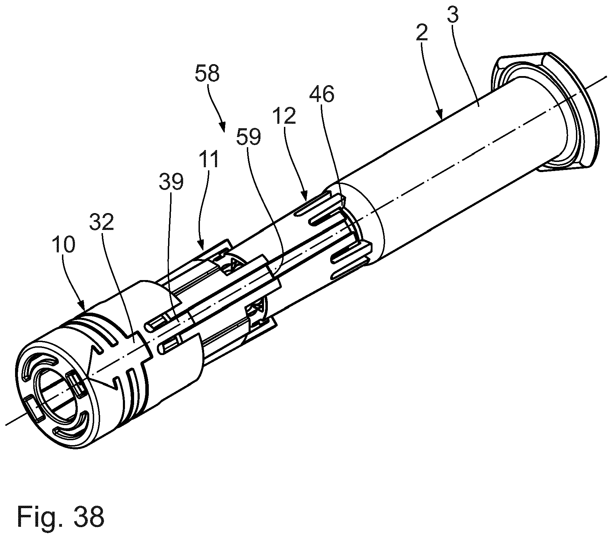

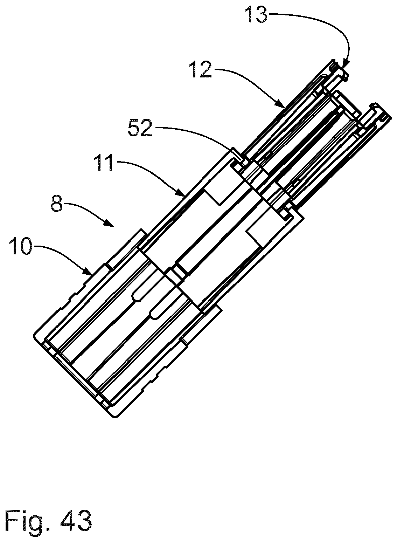

Beim Überführen der Stechschutzeinrichtung 8 von der Injektionsstellung in die Schutzstellung rücken einerseits die Rasthaken 52 der mittleren Teleskophülse 11 aus den Ausnehmungen 61 der Verbindungs-Teleskophülse 12 und andererseits die Rasthaken 52 der äußeren Schutz-Teleskophülse 10 aus den Gegenausnehmungen 59 der mittleren Teleskophülse 11 aus. Die Rasthaken der Teleskophülsen 10, 11 laufen dann axial längs den jeweiligen Führungsbahnen 60 der Teleskophülsen 11 und 12, bis die Rasthaken 52 der äußeren Schutz-Teleskophülse 10 in Ausnehmungen 63 einrücken, die an den Gegenausnehmungen 59 gegenüberliegenden Enden der Führungsbahnen 60 ausgeführt sind. In der Schutzstellung rücken weiterhin die Rasthaken 52 der mittleren Teleskophülse 11 in Ausnehmungen 63 ein, die an den Ausnehmungen 61 gegenüberliegenden Enden in den Führungsbahnen 60 der Verbindungs-Teleskophülse 12 ausgeführt sind. Somit ist die teleskopierte Schutzstellung nach Fig. 38 oder 43 erreicht.

## Patentansprüche

1. Medizinische Injektionsvorrichtung (1; 33; 41; 56; 58)
- mit einer Injektionseinrichtung (2), umfassend
-- einen Behälter (3) für ein zu injizierendes Medium,
-- eine Injektionskanüle (4), die mit dem Behälter (3) kommuniziert,
- mit einem Mündungs- und Verbindungsabschnitt (6), der mit einem kanülenseitigen Ende des Behälters (3) über eine Steckverbindung (7) verbunden ist,
- eine Schutzkappe (5) für die Injektionskanüle (4), die mit dem Mündungs- und Verbindungsabschnitt (6) an einem kanülenseitigen Ende des Behälters (3) lösbar verbunden ist,
- eine Verdrehschutzeinrichtung (8),
-- die drehfest mit dem Mündungs- und Verbindungsabschnitt (6) verbunden ist und
-- die den Mündungs- und Verbindungsabschnitt (6) in Form einer Hülse außen umgibt,
- wobei die Verdrehschutzeinrichtung (8) über einen Formschluss-Adapter (13) mit der Injektionseinrichtung (2) verbunden ist.

2. Injektionsvorrichtung nach Anspruch 1, **dadurch gekennzeichnet, dass** die Verdrehschutzeinrichtung (8) die Steckverbindung (7) axial überdeckt.

3. Injektionsvorrichtung nach Anspruch 1 oder 2, **dadurch gekennzeichnet, dass** der Formschluss-Adapter (13) mit dem Mündungs- und Verbindungsabschnitt (6) über eine Formschlussverbindung (27, 29) drehfest verbunden ist.

4. Injektionsvorrichtung nach Anspruch 3, **dadurch gekennzeichnet, dass** die Verdrehschutzeinrichtung (8) aufweist:
- mindestens eine Formschluss-Zunge (27) als Teil des Formschluss-Adapters (13), die zwischen zwei benachbarten, axial verlaufenden Umfangsrippen (29) des Mündungs- und Verbindungsabschnitts (6) aufgenommen ist,
- mindestens ein Niederhaltemittel (12; 48) zum Halten der Formschluss-Zungen (27) zwischen den Umfangsrippen (29).

5. Injektionsvorrichtung nach Anspruch 4, **dadurch gekennzeichnet, dass** die mindestens eine Formschluss-Zunge (27) einstückig an einem Tragring (28) des Formschluss-Adapters (13) angeformt ist, wobei der Tragring (28) auf den Mündungs- und Verbindungsabschnitt (6) aufschiebbar und mit diesem verrastet ist.

6. Injektionsvorrichtung nach Anspruch 4 oder 5, **dadurch gekennzeichnet, dass** das Niederhaltemittel mindestens eine innere Axialrippe (48) aufweist, die mit einem Niederhalte-Gegenstück (49) am Formschluss-Adapter (13) zum Niederhalten mindestens einer Formschluss-Zunge (27) zusammenwirkt.

7. Injektionsvorrichtung nach einem der Ansprüche 1 bis 6, **dadurch gekennzeichnet, dass** mindestens ein Bauteil (10; 36) der Verdrehschutzeinrichtung (8) als Mehrkomponenten-Spritzgussteil ausgeführt ist.

8. Injektionsvorrichtung nach Anspruch 7, **dadurch gekennzeichnet, dass** mindestens ein Mehrkomponenten-Spritzguss-Bauteil der Verdrehschutzeinrichtung (8) als äußere Hülse (10) ausgeführt ist, die mindestens einen Griffabschnitt (55) und mindestens einen Tragkörper (54) aufweist, wobei der Tragkörper (54) einerseits und der mindestens eine Griffabschnitt (55) andererseits als unterschiedliche Spritzguss-Komponenten des Mehrkomponenten-Spritzguss--Bauteils (10) ausgeführt sind.

9. Injektionsvorrichtung nach Anspruch 7 oder 8, **dadurch gekennzeichnet, dass** die Verdrehschutzeinrichtung (8) aufweist:
- mindestens ein Formschluss-Element (37), das zwischen zwei benachbarten, axial verlaufenden Umfangsrippen (29) des Mündungs- und Verbindungsabschnitts (6) aufgenommen ist,
- wobei das Formschluss-Element (37) an einem Formschluss-Element-Tragkörper (36) angeformt ist,
- wobei der Formschluss-Element-Tragkörper (36) einerseits und das mindestens eine Formschluss-Element (37) andererseits als unterschiedliche Spritzguss-Komponenten des MehrkomponentenSpritzguss-Bauteils (36) ausgeführt sind.

## Claims

1. Medical injection device (1; 33; 41; 56; 58) comprising
- an injection unit (2) including
-- a container (3) for a medium to be injected;
-- an injection cannula (4) which communicates with the container (3);
- a port and connection portion (6) which is connected, via a plug-in connection (7), to a near-cannula end of the container (3);
- a protective cap (5) for the injection cannula (4) which is detachably connected to the port and connection portion (6) at a near-cannula end of the container (3);
- an anti-rotation device (8)
-- which is non-rotationally connected to the port and connection portion (6); and
-- which surrounds the outside of the port and connection portion (6) in the manner of a sleeve
- wherein the anti-rotation device (8) is connected to the injection unit (2) via a positive-fit adapter (13).

2. Injection device according to claim 1, **characterised in that** the anti-rotation device (8) axially covers the plug-in connection (7).

3. Injection device according to claim 1 or 2, **characterised in that** the positive-fit adapter (13) is non-rotationally connected to the port and connection portion (6) via a positive-fit connection (27, 29).

4. Injection device according to claim 3, **characterised in that** the anti-rotation device (8) comprises:
- at least one positive-fit latch (27) which is part of the positive-fit adapter (13) and is received between two adjacent, axially extending peripheral ribs (29) of the port and connection portion (6);
- at least one hold-down means (12; 48) which holds the positive-fit latches (27) between the peripheral ribs (29).

5. Injection device according to claim 4, **characterised in that** the at least one positive-fit latch (27) is formed in one piece with a carrier ring (28) of the positive-fit adapter (13), wherein the carrier ring (28) is push-fittable onto the port and connection portion (6) and is snaplocked therewith.

6. Injection device according to claim 4 or 5, **characterised in that** the hold-down means has at least one inner axial rib (48) which interacts with a counter hold-down means (49) at the positive-fit adapter (13) in order to hold down at least one positive-fit latch (27).

7. Injection device according to one of claims 1 to 6, **characterised in that** the at least one component (10; 36) of the anti-rotation protection (8) is configured as a multi-component injection-moulded part.

8. Injection device according to claim 7, **characterised in that** the at least one multi-component injection-moulded part of the anti-rotation device (8) is configured as an outer sleeve (10) which has at least one grip portion (55) and at least one carrier body (54), wherein the carrier body (54) on the one hand and the at least one grip portion (55) on the other are configured as different injection-moulded components of the multi-component injection-moulded part (10).

9. Injection device according to claim 7 or 8, **characterised in that** the anti-rotation device (8) has:
- at least one positive-fit member (37) which is received between two adjacent, axially extending peripheral ribs (29) of the port and connection portion (6);
- wherein the positive-fit member (37) is formed at a positive-fit member carrier body (36);
- wherein the positive-fit member carrier body (36) on the one hand and the at least one positive-fit member (37) on the other are configured as different injection-moulded components of the multi-component injection-moulded part (36).

## Revendications

1. Dispositif médical d'injection (1 ; 33 ; 41 ; 56 ; 58)
- avec un équipement d'injection (2), comprenant
-- un récipient (3) pour un fluide à injecter,
-- une canule d'injection (4) qui communique avec le récipient (3),
- avec une section d'embouchure et de connexion (6) raccordée par une connexion à fiche (7) à une extrémité côté canule du récipient (3),
- un capuchon de protection (5) pour la canule d'injection (4), raccordée de manière amovible à la section d'embouchure et de connexion (6) sur une extrémité côté canule du récipient (3),
- une sécurité anti-rotation (8),
-- raccordée solidairement en rotation à la section d'embouchure et de connexion (6) et
-- entourant extérieurement la section d'embouchure et de connexion (6) sous la forme d'un manchon,
- dans lequel la sécurité anti-rotation (8) est raccordée à l'équipement d'injection (2) au moyen d'un adaptateur à engagement positif (13).

2. Dispositif d'injection selon la revendication 1, **caractérisé en ce que** la sécurité anti-rotation (8) recouvre axialement la connexion à fiche (7).

3. Dispositif d'injection selon la revendication 1 ou 2, **caractérisé en ce que** l'adaptateur à engagement positif (13) est raccordé solidairement en rotation à la section d'embouchure et de connexion (6) par une connexion à engagement positif (27, 29).

4. Dispositif d'injection selon la revendication 3, **caractérisé en ce que** la sécurité anti-rotation (8) comprend :
- au moins une languette à engagement positif (27) en tant que partie de l'adaptateur à engagement positif (13), laquelle est logée entre deux nervures périphériques (29) contiguës et à extension axiale de la section d'embouchure et de connexion (6),
- au moins un moyen de maintien (12 ; 48) pour le maintien des languettes à engagement positif (27) entre les nervures périphériques (29).

5. Dispositif d'injection selon la revendication 4, **caractérisé en ce que** ladite au moins une languette à engagement positif (27) est formée d'un seul tenant sur une bague de support (28) de l'adaptateur à engagement positif (13), dans lequel la bague de support (28) est coulissante sur la section d'embouchure et de connexion (6) et enclenchée avec celle-ci.

6. Dispositif d'injection selon la revendication 4 ou 5, **caractérisé en ce que** le moyen de maintien comprend au moins une nervure axiale intérieure (48), laquelle coopère avec une pièce complémentaire de maintien (49) sur l'adaptateur à engagement positif (13) pour le maintien d'au moins une languette à engagement positif (27).

7. Dispositif d'injection selon l'une des revendications 1 à 6, **caractérisé en ce qu'**au moins un élément (10 ; 36) de la sécurité anti-rotation (8) est réalisé comme pièce moulée par injection à plusieurs composants.

8. Dispositif d'injection selon la revendication 7, **caractérisé en ce qu'**au moins un élément moulé par injection à plusieurs composants de la sécurité anti-rotation (8) est réalisé comme manchon extérieur (10) présentant au moins une partie de préhension (55) et au moins un corps de support (54), dans lequel le corps de support (54), d'une part, et ladite au moins une partie de préhension (55), d'autre part, sont réalisés comme composants de moulage différents de l'élément moulé par injection à plusieurs composants (10).

9. Dispositif d'injection selon la revendication 7 ou 8, **caractérisé en ce que** la sécurité anti-rotation (8) comprend :
- au moins un élément à engagement positif (37) logé entre deux nervures périphériques (29) contiguës et à extension axiale de la section d'embouchure et de connexion (6),
- dans lequel l'élément à engagement positif (37) est formé sur un corps de support (36) d'élément à engagement positif,
- dans lequel le corps de support (36) d'élément à engagement positif, d'une part, et ledit au moins un élément à engagement positif (37), d'autre part, sont réalisés comme composants de moulage différents de l'élément moulé par injection à plusieurs composants (36).
